# FASCICULE DE BREVET EUROPEEN

(11) **EP 2 334 831 B1**
(45) Date de publication et mention de la délivrance du brevet: **17.06.2015**
(21) Numéro de dépôt: 09740503.9
(22) Date de dépôt: 28.08.2009
(51) Int. Cl.: H01M 10/0568, B22F 9/14, C22B 4/00, C07D 233/00, C22B 5/00, H01M 10/0525, H01M 4/58, B22F 9/24

(54) **SEL D'ANION PENTACYCLIQUE ET SON UTILISATION COMME ÉLECTROLYTE**
SALZ MIT PENTACYCLISCHEM ANION UND VERWENDUNG DAVON ALS ELEKTROLYT
PENTACYCLIC ANION SALT AND USE THEREOF AS AN ELECTROLYTE

(30) Priorité: 29.08.2008 FR 0804769
(43) Date de publication de la demande: 22.06.2011
(60) Demande divisionnaire: 15166637.7
(73) Titulaire: Centre National de la Recherche Scientifique, 75016 Paris (FR); Université de Picardie Jules Verne, 80039 Amiens (FR); Universite De Technologie De Varsovie, 00664 Varsovie (PL); Universite De Rome "La Sapienza", 00185 Rome (IT)
(72) Inventeur: BUKOWSKA, Maria, PL-01615 Varsovie (PL); SZCZECINSKI, Przemyslaw, PL-01650 Varsovie (PL); WIECZOREK, Wladyslaw, PL-05500 Mysiadlo (PL); NIEDZICKI, Leszek, PL-00195 Varsovie (PL); SCROSATI, Bruno, I-00198 Rome (IT); PANERO, Stefania, I-00144 Rome (IT); REALE, Priscilla, I-04100 Latina (IT); ARMAND, Michel, F-75014 Paris (FR); LARUELLE, Stéphane, F-80470 Saveuse (FR); GRUGEON, Sylvie, F-60960 Feuquieres (FR)
(74) Mandataire: Goulard, Sophie
(86) Numéro de dépôt international: PCT/FR2009/051642
(87) Numéro de publication internationale: WO 2010/023413

(56) Documents cités:
- EP-A- 0 850 933
- DE-A1-102006 013 871
- US-A- 3 793 339
- BEGLAND, R. W. ET AL: "Hydrogen cyanide chemistry. VIII. New chemistry of diaminomaleonitrile. Heterocyclic synthesis" JOURNAL OF ORGANIC CHEMISTRY , 39(16), 2341-50 CODEN: JOCEAH; ISSN: 0022-3263, 1974, XP002521054
- NIEDZICKI L ET AL: "Modern generation of polymer electrolytes based on lithium conductive imidazole salts" JOURNAL OF POWER SOURCES, ELSEVIER SA, CH, vol. 192, no. 2, 15 juillet 2009 (2009-07-15), pages 612-617, XP026140144 ISSN: 0378-7753 [extrait le 2009-04-02]
- KOO I G ET AL: "Platinum nanoparticles prepared by a plasma-chemical reduction method" JOURNAL OF MATERIALS CHEMISTRY, THE ROYAL SOCIETY OF CHEMISTRY, CAMBRIDGE, GB, vol. 15, 1 janvier 2005 (2005-01-01), pages 4125-4128, XP002448492 ISSN: 0959-9428

## Description

La présente invention concerne une composition d'électrolyte contenant un sel et un solvant, particulièrement utile dans des batteries.

Les solutions d'électrolyte dans un milieu non aqueux, en particuliers non-protogénique et plus communément appelé « aprotique » sont d'une grande importance technologique, car elles permettent d'étendre le domaine de potentiel dans lequel une batterie peut fonctionner sans réaction parasite telle que la décomposition du solvant, ledit potentiel ne dépassant pas la valeur de 1,3 V dans l'eau.

Les milieux capables de dissoudre des sels sont principalement des solvants organiques polaires ou des polymères solvatants, en particulier ceux contenant des groupements éthers répartis dans un chaîne macromoléculaire dont l'architecture peut être linéaire ou ramifiée, de type peigne, possédant ou non des noeuds de réticulation. Les polyéthers possédant les unités de répétition - CH₂CH₂O - sont particulièrement appréciées pour leur pouvoir solvatant élevé.

On connaît aussi les liquides ioniques qui sont des sels fondus à basse température, constitués par au moins un cation à charge délocalisée, tel que l'éthylméthyl imidazolium (EMI), le méthyl-propyl pyrrolidinium, le diéthyl-méthyl-2-méthoxyéthyl ammonium, et par un anion, possédant de préférence lui aussi un charge délocalisée sur un volume important pour diminuer les interactions entre les cations et les anions et pour permettre ainsi d'atteindre des températures de solidification basses.

Les solutés destinés à amener la conductivité de type ionique requise pour les électrolytes sont choisis parmi les sels métalliques et parmi les sels dits « ium » obtenus par engagement d'un doublet électronique libre d'un ou plusieurs éléments tels que N, O, S, P, As ou I avec un proton ou un radical organique pour former un cation. On peut citer les ions ammonium, phosphonium, sulfonium, iodonium, pyridinium, imidazolium, oxazolium et thiazolium. Parmi les métaux, une importance particulière est donnée aux sels alcalins et alcalino-terreux, notamment aux sels de lithium. L'ion lithium possède effectivement une électrochimie très riche, permettant de constituer des batteries à haute densité d'énergie qui sont très importantes dans la technologie actuelle. Comme autres applications des électrolytes non aqueux, on peut citer les systèmes électrochromes et les supercapacités.

Les anions qui servent de contre-charge aux cations sont choisis parmi ceux qui présentent un charge négative délocalisée, car les électrolytes aprotiques ne peuvent former des liaisons hydrogène avec les charges négatives, et la délocalisation est le seul moyen d'obtenir, une dissociation appréciable dans ces conditions. Parmi les anions les plus connus, on peut citer ClO₄⁻, BF₄⁻, PF₆⁻, AsF₆⁻, SbF₆⁻. L'anion ClO₄⁻ peut former des mélanges explosifs Les anions dérivés de As et Sb sont toxiques et rares. L'anion BF₄⁻ est relativement peu dissocié. Les sels de l'anion LiPF₆ sont les sels le plus utilisés dans les générateurs au lithium, malgré des inconvénients majeurs : i) ils sont très facilement hydrolysables, libérant HF qui est toxique et corrosif vis-à-vis des matériaux d'électrode. HF libère des cations (Mn, Fe...) de l'électrode positive, et leur permet de migrer vers l'électrode négative où ils sont réduits (Mn°, Fe°...), ce qui augmente notablement l'impédance interfaciale de cette électrode, en diminuant la puissance disponible et la durée de vie ; ii) l'équilibre acido-basique LiPF₆ ⇔ LiF + PF₅ libère un acide de Lewis très puissant capable d'induire une chimie carbo-cationique, destructrice en particulier pour les esters ou éthers qui peuvent constituer le solvant électrolytique ; iii) en cas de réaction non contrôlée, ("runaway reaction") avec fort échauffement, LiPF₆ peut agir comme agent fluorant en donnant des dérivés du monofluoroéthanol ou de l'acide monofluoroacétique qui sont excessivement toxiques.

On connaît aussi un anion de coordination sans fluor, en particulier le bis(oxalato)borate [B(C₂O₄)₂]⁻, qui met en oeuvre des éléments peux onéreux, mais dont le sel de lithium a une conductivité limitée. La rigidité de l'anion et sa taille importante lui donnent in diagramme de phase défavorable dans les électrolytes courants contenant du carbonate d'éthylène (mauvaise conductivité à basse température). De plus, cet anion a une stabilité très limitée en oxydation à haute température (65 °C), ce qui induit des problèmes d'autodécharge et de dégagement gazeux.

On connaît d'autres anions qui présentent une grande stabilité électrochimique ainsi que des conductivités élevées aussi bien dans les liquides que les polymères. Parmi ceux-ci, les anions capables de former un liquide ionique sont les plus performants. La famille principale est celle des sulfonimides [(R_{F}SO₂)₂N]⁻, dont le représentant le plus important correspond à R_{F} = CF₃ (TFSI). Les inconvénients de ces sels sont dus d'une part à l'absence de passivation de l'aluminium au dessus de 3,6 V vs. Li⁺ : Li° lorsque les sels sont utilisés dans des batteries ou des supercapacités qui ont une électrode dont le collecteur de courant en en aluminium. Un autre inconvénient est le coût de préparation élevé relié au prix du synthon CF₃SO₂. L'anion [(FSO₂)₂N]⁻ aurait un comportement plus favorable vis-à-vis de la corrosion de l'aluminium, mais sa préparation est très onéreuse et la stabilité du sel de lithium est limitée (130°C). D'une manière générale, il semble que la corrosion de l'aluminium soit inévitable au-dessus de 3,6 volts lorsque l'électrolyte contient un sel d'un anion covalent car il peut se former un sel d'aluminium soluble (tel que par exemple le sel de TFSI[(CF₃SO₂)₂N]₃Al qui est stable et très soluble) qui ne permet pas de passiver la surface du métal. En revanche, un anion de coordination tel que PF₆⁻ ne forme pas (PF₆)₃Al, mais le sel AlF₃ qui est insoluble et passivant.

D'autres anions, dits « Hückel anions-», sont basés sur la transposition de la règle de Hückel (4n + 2) qui prédit la stabilité des systèmes aromatique, appliquée aux cycles à cinq atomes dont la charge négative est fortement favorisée. Le plus connus de ces anions est le 4,5-dicyano-triazole (DCTA) : 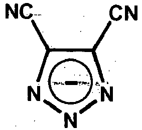 Cet anion purement covalent peut-être considéré comme ayant une configuration à 6 électrons « π » ou une configuration à 10 électrons « π » selon que les électrons des liaisons C≡N des groupements nitrile sont pris en compte ou non, chacune de ces configurations étant stable. Les sels du DCTA sont stables thermiquement jusqu'à 300°C. De plus, l'anion DCTA ne contient pas de fluor et il est facilement fabriqué à partir d'un précurseur industriel, le diamino-maléonitrile (DAMN) : 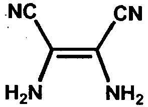 Cet anion a cependant pour inconvénient une conductivité relativement modeste de son sel de lithium (2,9 mS.cm⁻¹ dans EC-DMC 50/50) et surtout un potentiel d'oxydation de 3,7 V vs. Li⁺ : Li° qui limite son utilisation d'une manière rédhibitoire pour les matériaux d'électrode de type oxydes de métaux de transition LiₓT^{M}O₂ (0 ≤ x ≤ 1) avec T^{M} = Mn, Ni, Co, le phosphate de manganèse LiMnPO₄ ou ses solution solides avec le phosphate de fer LiMn_{1-y}Fe_{y}PO₄ (0 ≤ y < 1). Même pour le phosphate de fer (y = 1) dont le potentiel est 3,5 V vs. Li⁺ : Li°, la marge de sécurité en fin de charge de l'électrode est trop faible.

On connaît également, notamment par EP-0 850 933-A, une composition d'électrolyte comprenant des sels d'anions répondant à la formule : 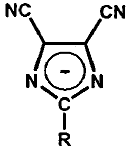 dans laquelle R est un groupe électroattracteur, par exemple un groupe perfluoroalkylsulfonyle ou un groupe perfluoroalkylcarbonyle. Cependant, malgré le pouvoir attracteur élevé du groupement R, la présence d'oxygène (C=O et O=S=O) qui donne de très fortes interactions avec les cation, limite la dissociation. En outre, les groupements C=O ou S=O sont en conjugaison avec le cycle, et le nombre d'électrons "π"» est un multiple de 4. Il en résulte que les systèmes sont "antiaromatiques" et ils qu'ils ont donc une stabilité en oxydation et en réduction plus faible. De plus, la préparation de ce type de composé est très difficile et ne peut se faire en une seule étape à partir du DAMN.

La synthèse du 2-trifluorométhyl-4,5-dicyanoimidazole est décrite par M. Bukowska, et al. [Polish J. Chem. 78, 417-422 (2004)]. Le sel de lithium correspondant peut être obtenu par réaction avec le carbonate de lithium.

Le but de la présente invention est de fournir une composition d'électrolyte contenant un sel et un solvant, pouvant être utilisée dans les dispositifs électrochimiques au lithium, ledit sels étant stable à des températures élevées et à des potentiels supérieurs à 4 V vs. Li⁺ : Li°.

L'invention a donc pour objet une composition d'électrolyte contenant un sel (i.e. un composé ionique) et un solvant, caractérisée en ce que le sel a un cation M de valence m (1≤m≤3) et m anions répondant à la formule : 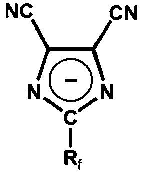 dans laquelle R_{f} est un groupe -CFZ'Z" dans lequel :
- Z' est F ou un groupe perfluoroalkyle ayant de 1 à 3 atomes de carbone,
- Z" est un groupe H, F, Cl, un groupe alcoxy éventuellement fluoré ou perfluoré ayant de 1 à 5 atomes de carbone, un groupe oxaalcoxy éventuellement fluoré ou perfluoré ayant de 1 à 5 atomes de carbone, ou un groupe alkyle éventuellement fluoré ou perfluoré ayant de 1 à 5 atomes de carbone.
- le cation M étant un cation inorganique, en particulier choisi parmi les cations de métal alcalin et les cations de métal alcalino-terreux ou un cation ammonium, de préférence un ion lithium ou un ion sodium.

A titre d'exemples, on peut citer les groupes R_{f} suivants : CF₂H, CF₂Cl, C₂F₅, CF₂CF₂H, C₃F₇, C₄F₉CF₂OCH₃, CF₂OC₂H₅, CF₂OC₂H₄OCH₃, CF₂OC₂H₄OC₂H₅, CF₂OCH₂OCF₃, CF(CF₃)OCH₃, CF(CF₃)OC₂H₅, CF(CF₃)OC₂H₄OCH₃, CF(CF₃)OC₂H₄OC₂H₅ et CF(CF₃)OCH₂CF₃.

Le solvant est choisi parmi les solvants organiques liquides éventuellement gélifiés par un polymère, les polymères solvatants éventuellement plastifiés par un solvant liquide, les mélanges de polymère non solvatant et d'un liquide polaire ou d'un liquide ionique, et les liquides ioniques

Par solvant organique liquide, on entend un liquide polaire ou un mélange de liquides polaires susceptible de dissoudre le sel de la composition d'électrolyte de la présente invention. Comme exemples de liquides polaires, on peut citer notamment les éthers linéaires et les éthers cycliques, les esters, les nitriles, les dérivés nitrés, les amides, les sulfones, les sulfolanes, les alkylsulfamides et les hydrocarbures partiellement halogénés. Les solvants particulièrement préférés sont le diméthoxyéthane, le glyme, le tétrahydrofurane, le dioxane, le méthyltétrahydrofurane, le formiate de méthyle ou d'éthyle, le carbonate de propylène ou d'éthylène, les carbonates d'alkyles (notamment le carbonate de diméthyle, le carbonate de diéthyle et le carbonate de méthylpropyle), les butyrolactones, l'acétonitrile, le benzonitrile, le nitrométhane, le nitrobenzène, le diméthylformamide, le diéthylformamide, la N-méthylpyrrolididone, la diméthylsulfone, la tétraméthylène sulfone, le diméthylsufoxyde et les tétraalkylsulfonamides ayant de 5 à 10 atomes de carbone ou leur mélanges.

Par liquide ionique, on entend un sel ou un mélange de sels d'un cation inorganique ou organique ayant une température de fusion ≤ 100 °C. Comme exemples de liquides ioniques, on peut citer notamment les sels d'un cation organique et d'un anion choisi dans le groupe constitué par BF₄⁻, CF₃SO₃⁻, TFSI, FSI, C(CN)₃⁻ et N(CN)₂. On peut en outre citer les composés ioniques comprenant un cation M de valence m (1≤m≤3) et m anions répondant à la formule : 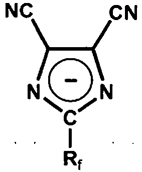 dans laquelle R_{f} est un groupe -CFZ'Z" dans lequel :
- Z' est F ou un groupe perfluoroalkyle ayant de 1 à 3 atomes de carbone,
- Z" est un groupe H, F, Cl, un groupe alcoxy éventuellement fluoré ou perfluoré ayant de 1 à 5 atomes de carbone, un groupe oxaalcoxy éventuellement fluoré ou perfluoré ayant de 1 à 5 atomes de carbone, ou un groupe alkyle éventuellement fluoré ou perfluoré ayant de 1 à 5 atomes de carbone.
- le cation M étant un cation organique ou organométallique, en particulier les sels d'un cation ammonium, phosphonium, sulfonium, iodonium, pyridinium, imidazolium, pyrazolium, acetamidium, oxazolium, thiazolium, pyrrolidinium ou piperidinium, plus particulièrement les sels d'un cation choisi parmi les cations éthyl-méthyl-imidazolium, butyl-méthyl-imidazolium, méthyl-propyl-pyrrolidinium, méthyl-butyl-pyrrolidinium, méthyl-propyl-piperidinium, butyl pyridinium, (2-méthoxy-éthyl)-triéthyl-ammonium, et hexyl-trimethyl ammonium.

On peut également citer les cations organiques qui répondent aux formules suivantes, dans lesquelles :
- R¹ à R³⁷ représentent chacun H, ou un groupe alkyle, aryle, ou oxaalkyle de 1 à 20 atomes de carbone ;
- R⁵ à R¹³ représentent chacun un groupe aryle, alkylaryle, ou dialkylamino R³⁷R³⁸N dans lesquels les groupes R³⁷ et R³⁸ sont des groupes alkyle ayant de 1 à 20 atomes de carbone ;
- ou bien deux groupes R portés par des atomes de carbone adjacents forment ensemble un biradical formant un cycle aliphatique ou aromatique.

| | | | |
|---|---|---|---|
| 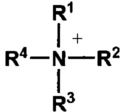 | 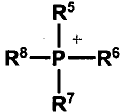 | 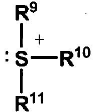 | 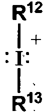 |
| ammonium | phosphonium | Sulfonium | iodonium |
| 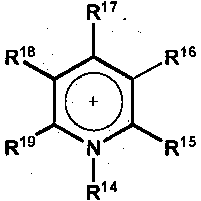 | 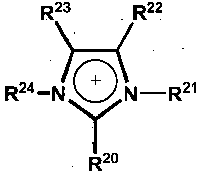 | 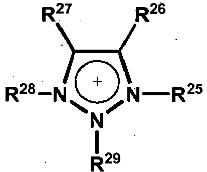 | 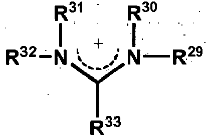 |
| pyridinium | imidazolium | pyrazolium | acetamidinium |
| 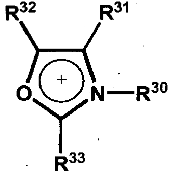 | 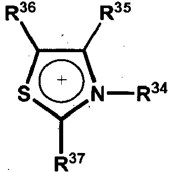 | 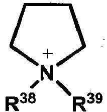 | 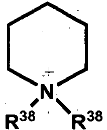 |
| oxazolium | thiazolium | pyrrolidinium | piperidium |

Les ions pyrrolidinium et pipéridium sont deux exemples importants d'ammonium quaternaire dans lequel deux substituants de l'azote forment ensemble un cycle.

Dans un mode de réalisation particulier, le composé ionique du liquide ionique comprend une partie polycationique organique associée au nombre d'anions requis pour assurer l'électroneutralité du composé. La partie polycationique comprend au moins deux unités récurrentes qui portent chacune un groupe cationique. Selon une variante, l'unité récurrente de la partie polycationique peut être une unité portant un groupe latéral cationique, par exemple l'un des cations ci-dessus dans lequel l'un des groupes R est un biradical de liaison avec l'unité récurrente formant la chaîne du groupe polycationique. Selon une autre variante, les groupes cationiques font partie de la chaîne du groupe polycationique, deux substituants R sur un groupe cationique étant des biradicaux qui forment une liaison avec des groupes cationiques adjacents.

Un cation organométallique peut être choisi parmi les ions ferricinium, titanocenium et zirconocénium. On peut citer en particulier le cation ferricinium [C₅H₅)₂Fe]⁺, le cation titanocénium [C₅H₅)₂Ti]²⁺ et le cation zirconocénium [C₅H₅)₂Zr]²⁺.

Par polymère solvatant, on entend un polymère possédant en quantité suffisante des fonctions capables de former un complexe avec les sels de métaux décrits plus haut. Un tel polymère peut être choisi parmi les polymères solvatants, réticulés ou non, portant ou non des groupes ioniques greffés. A titre d'exemple de polymères solvatants, on peut citer les polyéthers de structure linéaire, peigne ou à blocs, formant ou non un réseau, à base de poly(oxyde d'éthylène), ou les copolymères contenant le motif oxyde d'éthylène ou oxyde de propylène ou allylglycidyléther, les polyphosphazènes, les réseaux réticulés à base de polyéthylène glycol réticulé par des isocyanates ou les réseaux obtenus par polycondensation et portant des groupements qui permettent l'incorporation de groupements réticulables. On peut également citer les copolymères à blocs dans lesquels certains blocs portent des fonctions qui ont des propriétés rédox..

La concentration en composé ionique d'une composition d'électrolyte liquide selon l'invention, dans laquelle le solvant est du type solvant organique polaire ou du type liquide ionique, est comprise de préférence entre 10⁻³ mole/L et 3,5 moles/L.

Dans une composition d'électrolyte à solvant polymère dans laquelle le polymère est constitué d'unités récurrentes oxyalkylène, la concentration en composé ionique est de préférence telle que le nombre d'atomes d'oxygène (ou d'unités récurrentes) par mole de composé ionique soit entre 1 et 200.

D'une manière inattendue, les composés ioniques de la composition d'électrolyte de l'invention ont des propriétés très supérieures en termes de conductivité et de stabilité électrochimique, aux autres anions de Hückel conventionnels. En particulier, les anions des sels de la composition d'électrolyte de l'invention ont une stabilité anodique supérieure à 4,5 V vs. Li⁺/Li°. Cette résistance à l'oxydation est tout à fait exceptionnelle si l'on considère que les anions de type DCTA et les anions des sels de la composition d'électrolyte de l'invention sont dérivés des acides [ZO₂]⁻ par condensation sur le DAMN selon le schéma réactionnel suivant : 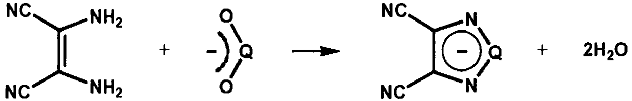 Q étant N pour les composés de type DCTA de l'art antérieur et CR_{f} pour les composés de la composition d'électrolyte de la présente invention, notamment CF₃C.

On peut donc comparer les acidités et potentiels d'oxydation du DCTA et des anions des sels de la composition d'électrolyte de l'invention en comparant celle de leur précurseur acide, c'est-à-dire l'acide nitreux NO₂⁻ dont le pKₐ est 3,4 (pour le composé de type DCTA) et l'acide trifluoroacétique CF₃CO₂⁻, dont le pKₐ est de 0,23 à 25°C pour le composé ionique de la composition d'électrolyte de l'invention dans lequel R_{f} est CF₃. Au vu de ces valeurs, on pourrait supposer que le potentiel d'oxydation de l'anion du sel de la composition d'électrolyte de l'invention R_{f} = CF₃ serait décalé de 56 x (3,4-0,23) = 178 mV vers les potentiels anodiques soit à ≈ 4V vs Li⁺ : Li°. Ce potentiel ne serait pas suffisant pour assurer le fonctionnement électrochimique des oxydes de type LiₓT^{M}O₂ (0 ≤ x ≤ 1) ni du phosphate de manganèse LiMnPO₄ ou de LiMn_{1-y}Fe_{y}PO₄ (0 ≤ y < 1). On considère qu'une stabilité anodique de 4,3 V de l'électrolyte est requise pour des générateurs électrochimiques utilisant ces matériaux de cathode.

Or les cycles de voltamétrie effectués pour divers composés ioniques de la composition d'électrolyte de l'invention dans lesquels R_{f} est respectivement -CF₃, -CF₂Cl, -CF₂OCH₃, -CF₂OCF₃, -C₂F₅, -C₂F₄H, -C₃F₇, ou -C₄F₉, montrent que le potentiel d'oxydation des composés ioniques de la composition d'électrolyte de l'invention est supérieur à 4,3 V, bien au-dessus de ce que prévoyait la théorie.

D'une manière tout aussi surprenante, les sels de lithium des composés ioniques de la composition d'électrolyte de l'invention ne corrodent pas l'aluminium à des potentiels inférieurs à 4,6 V, ce qui en fait d'excellents candidats pour des batteries au lithium dans lesquelles le collecteur de courant est une feuille d'aluminium, qui a des avantages de poids et de coût.

Un avantage intéressant de plusieurs composé ioniques de la composition d'électrolyte de l'invention est le prix peu élevé des produits de départ. Pour R_{f} = CF₃, l'acide trifluoroacétique est un produit industriel qui dérive de la préparation des fluides réfrigérants (tels que CF₃CH₂F par exemple).

Les compositions d'électrolyte de la présente invention sont particulièrement utiles dans les dispositifs électrochimiques qui fonctionnent par échange d'ions lithium entre une anode et une cathode. Il s'agit notamment de batteries au lithium, de batteries lithium-ion, de supercondensateurs et de dispositifs électrochromes. Le composé ionique utilisé est alors de préférence un sel de lithium.

Un composé ionique de la composition d'électrolyte de l'invention peut être obtenu par un procédé comprenant :
- une première étape consistant à préparer un acide 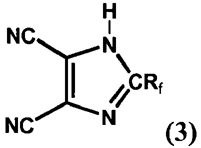
- une deuxième étape au cours de laquelle l'acide est transformé en sel du cation M.

Le composé (3) peut être obtenu par réaction entre le DAMN et un réactif apportant le groupe R_{f}.

Dans un 1 er mode de réalisation, la première étape est mise en oeuvre selon le schéma réactionnel suivant : 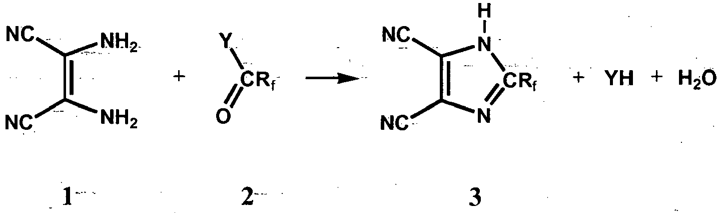 dans lequel R_{f} a la signification donnée ci-dessus et Y représente R_{f}C(=O)O, Cl, F, CF₃SO₃, OCH₃, OC₂H₅, OCH₂CF₃, OC₆H₄NO₂ (nitrophényle), un groupe imidazoyle ou un groupe succinimidyloxy.

Dans un 2^{ème} mode de réalisation, la première étape est effectuée à partir d'un aldéhyde (4) O=CHR_{f}, selon le schéma réactionnel suivant : 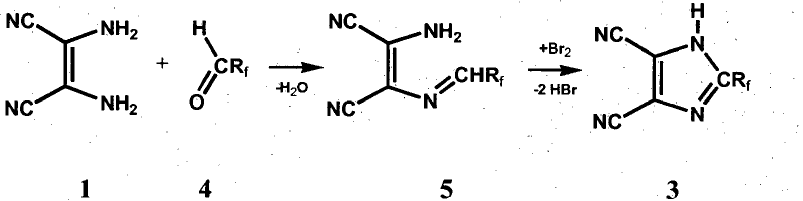 R_{f} a la signification donnée ci-dessus.

Dans un 3^{ème} mode de réalisation, la 1^{ère} étape du procédé est mise en oeuvre à partir d'un acétal (6), selon le schéma réactionnel suivant:

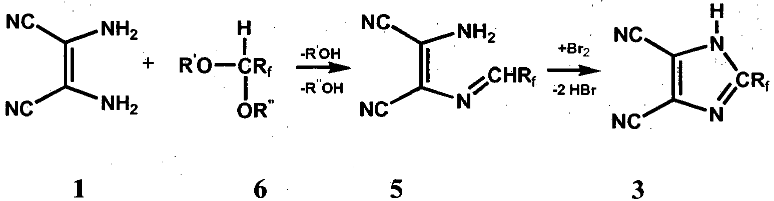

Le 2^{ème} et le 3^{ème} modes de réalisation sont intéressants lorsque l'aldéhyde R_{f}CHO ou ses acétals R_{f}CH(OH)(OR'), R_{f}CH(OH)₂ et R_{f}CH(OR')(OR") sont disponibles dans le commerce, par exemple lorsque R' et R" sont CH₃, C₂H₅, n-C₃H₇, ou i-C₃H₇.]

Dans les procédés mettant en oeuvre un aldéhyde ou un acétal, le brome peut être remplacé par un autre agent oxydant de force similaire pour la cyclisation du composé 5. Par exemple, le brome peut être remplacé par le chlore à basse température ou par une imide telle que la N-chloro succinimide ou la N-bromo succinimide, un hypochlorite,- ou le sel de sodium de l'acide N,N-dichlorocyanurique.

Le composé acide (3) obtenu à la fin de la 1^{ère} étape peut être transformé en sel du cation M souhaité, par les méthodes et techniques connues de l'homme de métier. On peut citer notamment la réaction d'un composé (3) avec un carbonate, un hydrogéno carbonate, un acétate, un méthylcarbonate, ou un hydroxyde du cation M. Lorsque le cation M est un cation organique, la transformation peut être faite en deux étapes : transformation, du composé acide 3 en sel de sodium ou de potassium, puis réaction du sel de sodium ou de potassium avec une quantité stoechiométrique d'un composé du cation organique (par exemple le chlorure, le bromure, un alkylsulfate), dans un solvant aprotique dans lequel le sel de Na ou de K est insoluble, par exemple l'acétonitrile

Un composé 2 dans lequel le groupe R' de R_{f} est F, le groupe R" et le groupe Y représentent chacun un groupe alcoxy ou oxaalxoxy OZ¹ (désigné par composé 2'), peut être obtenu par réaction d'un composé HOZ¹ (8) avec le tétrafluorooxirane(7), selon le schéma réactionnel 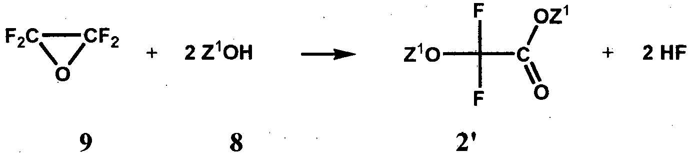 Les composés 2' concernés sont notamment ceux dans lesquels Z¹ est un alkyle ou un alkyloxyalkyle, lesdits groupes étant éventuellement fluorés ou perfluorés. A titre d'exemple de groupe Z¹, on peut citer les groupes OCH₃, OC₂H₅, OC₂H₄OCH₃, OC₂H₄OC₂H₅, OCH₂CF₃, et OCF₃.

Une modification de la réaction en milieu basique permet, à partir de l'anion CF₃O⁻, d'obtenir les dérivés de CF₃OCF₂CO₂H". Cette variante permet de préparer un composé dans lequel le groupe R_{F} est CF₂OCF₃.

Un composé 2 dans lequel le groupe R' de R_{f} est CF₃, le groupe R" et le groupe Y représentent chacun un groupe alcoxy ou oxaalxoxy OZ² (désigné par composé 2") peut être obtenu par réaction d'un composé HOZ² (8) avec le l'oxirane (10), selon le schéma réactionnel

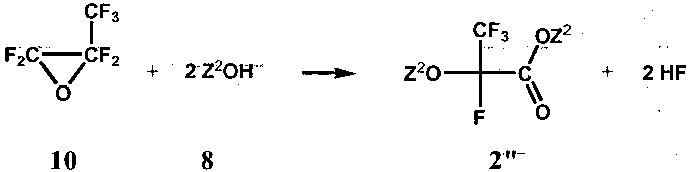

Les composés 2" concernés sont notamment ceux qui contiennent un groupe Z² tel que OCH₃, OC₂H₅, OC₂H₄OCH₃, OC₂H₄OC₂H₅ ou OCH₂CF₃,

Les composés O=C(R_{f})Y dans lesquels le groupe R_{f} est CF₃, CF₂H, CF₂Cl, C₂F₅, HC₂F₄, C₃F₇, ou C₄F₉, sont disponibles dans le commerce sous forme d'acides, d'anhydrides ou d'esters à partir desquels la formation du cycle du dicyanoimidazole est possible.

Les composés 2 répondant à la formule O=C(R_{f})Y peuvent être préparés à partir de l'acide O=C(R_{f})OH correspondant, par réaction avec un réactif approprié, par des procédés connus de l'homme de métier. Par exemple le réactif est un agent chlorurant (par exemple SOCl₂) si Y est Cl, un carbonyl imidazole si Y est un groupe imidazole, le carbonate de nitrophényle si Y est un groupe nitrophényle, ou le carbonate de succinimidoyie si Y est un groupe succinimidyloxy.

La présente invention est illustrée par les exemples suivants, auxquels elle n'est cependant pas limitée.

### Exemple 1

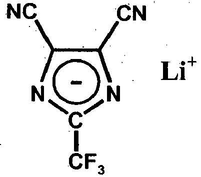

On a introduit 2,42 g d'anhydride trifluoroacétique dans un réacteur contenant une solution de 1,14 g de DAMN dans 11 mL de dioxane. Le mélange a été maintenu sous argon et agité sous reflux jusqu'à disparition complète des réactifs. Après élimination sous vide du solvant et de l'acide trifluoroacétique, on a dissous le résidu solide dans 50 mL d'éther. La solution d'éther a été extraite 4 fois avec une suspension à 1 g de carbonate de lithium dans 90 mL d'eau, puis la solution aqueuse du sel de lithium a été lavée à l'éther. Après élimination de l'eau dans un évaporateur rotatif, le résidu foncé a été séché sous vide à 100°C. Le solide de couleur foncée a ensuite été extrait à l'acétonitrile (4×10 mL) et la solution résultante a été filtrée. L'acétonitrile a ensuite été éliminé et le sel brut a été purifié par chromatographie sur alumine en utilisant comme éluant un mélange acétonitrile/benzène 2/1. Après séchage, on a obtenu 1,45 g (rendement 71%) de 2-trifluoromethyl-4,5-dicyano-imidazole sous forme d'un solide incolore. Le sel de lithium (LiTDCI) est obtenu sous forme d'un di-solvate après recristallisation. Le produit pur est obtenu par traitement sous vide à 150 °C.

On a préparé plusieurs échantillons d'électrolyte polymère en dissolvant 680 mg de poly(oxyde d'éthylène) dont la masse molaire M_{w} est de 10⁵, 200 mg de poly(oxyde d'éthylène) dont la masse molaire M_{w} est de 5.10⁶, et LiTDCI dans 13 ml d'acétonitrile, sous agitation jusqu'à obtention d'une solution légèrement opalescente et visqueuse.

On a ainsi préparé trois échantillons en utilisant respectivement 180 mg, 320 mg et 240 mg de LiTDCI.

Chacune des solutions est coulée dans un anneau de verre de 50 cm de diamètre posé sur une plaque de verre recouverte de PTFE. Après évaporation de l'acétonitrile sous un flux d'air sec, on obtient un film élastique et transparent de complexe.

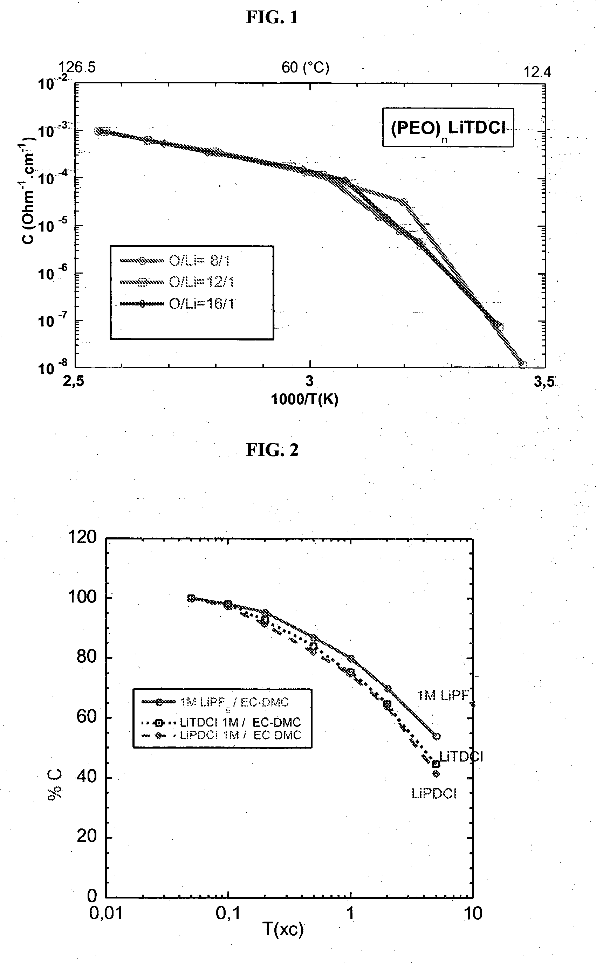

La conductivité de ces électrolytes a été mesurée en fonction de la température. La figure 1 représente la conductivité C (en ohm⁻¹.cm⁻¹) en fonction de la température exprimée en 1000/T(K). La concordance entre les courbes et les échantillons est données dans le tableau ci-dessous :

| Teneur en LiTDCI | Echantillon | courbe. |
|---|---|---|
| 480 mg | P(EO)₈LiTDCI, | O/Li=8/1 |
| 320 mg | P(EO)₁₂LiTDCI | O/Li=12/1 |
| 240 mg | P(EO)₁₆LiTDCI | O/Li=16/1 |

La conductivité de LiTDCE est comparable à celle de Li[CF₃SO₂)₂N] (LiTFSI), qui est le sel de référence pour la conductivité des électrolytes polymères.

### Exemple 2

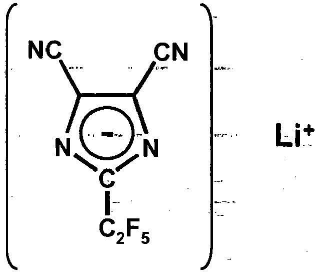

On a ajouté 10;5 mL (53,8 mmol) d'anhydride pentafluoropropionique dans une solution de 4,84 g (44,8 mmol) de diaminomaléonitrile dans 47 mL de dioxane. Le mélange a été chauffé au reflux sous argon jusqu'à disparition du précipité [vérifié par chromatographie en couche mince (TLC), environ 6 h]. Le mélange résultant a été mis sous vide pendant 1 h à 90°C, puis séché sur une rampe à vide secondaire pendant 1 h à 120° C pour éliminer le solvant et l'acide. Le résidu solde a été dissous dans 40 mL d'éther et la solution résultante a été extraite trois fois avec une suspension de 3 g (40,5 mmol) de carbonate de lithium dans 100 mL d'eau. La solution aqueuse de sel a été lavée deux fois par 50 mL d'éther. Ensuite on a ajouté à la solution aqueuse du charbon actif agissant comme décolorant, et la boue a été chauffée pendant 1 h. Après élimination du charbon actif par filtration sur un filtre en papier, la solution a été séchée sous vide pendant 2 h à 80°C. Ensuite, le résidu a été dissous dans l'acétonitrile anhydre et on a filtré à nouveau le résidu solide. La solution d'acétonitrile a été mise sous vide pendant 1 h à 90°C. Une double cristallisation dans un mélange acétonitrile/ benzène 1/1 donne des cristaux qui sont mis sous vide sur une rampe à vide secondaire pendant 4 h à 120°C. On a obtenu 5,12 g de cristaux incolores de 4,5-dicyano-2-(pentafluoroethyl)imidazole de lithium [LiPDCI] (rendement : 47,2%).

### Exemple 3

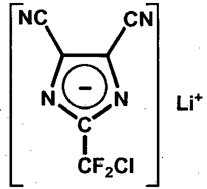

10,8 g de DAMN et 22 g d'anhydride chlorodifluoroacétique (ClF₂CO)₂O sont ajoutés dans 100 ml de diglyme et portés à reflux sous atmosphère protectrice d'azote et la réaction est poursuivie pendant 48-heures. Les produits de la réaction sont filtrés et traités avec 12 g de carbonate de sodium et la solution est évaporée. Le résidu solide est repris dans 80 ml d'eau et 25 g d'acide sulfamique. Le mélange de 2-chlorodifluoroethyl-4,5-imidazole et d'acide chlorodifluoroacétique sous-produit de la réaction sont extrait par trois portions de 50 ml d'éther. Les portions sont combinées et évaporée. Le 2-chlorodifluorométhyl-4,5-imidazole brut est purifié par sublimation sous vide primaire à 90°C dans un four Büchi.

Le sel de lithium est obtenu en faisant réagir 5 g de la forme acide de l'imidazole sur un léger excès stoechiométrique de carbonate de lithium (1,1 g) dans l'acétonitrile. La suspension est centrifugée et le sel de lithium. Li[CClF₂C₃N₂(CN)₂] est obtenu sous forme d'une poudre blanche hygroscopique.

### Exemple 4

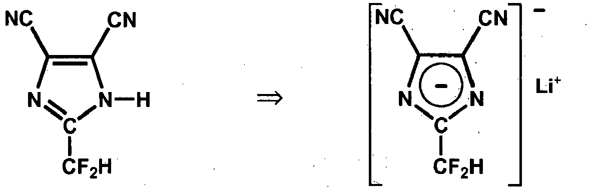

À 9,6 g d'acide difluoroacétique commercial dans 75 ml de diglyme sont ajoutés 16,2 g de carbonyl-di-imidazole commercial. Un dégagement de CO₂ se produit après quelques minutes. À la solution claire obtenue sont ajoutés 10,8 g de DAMN. La réaction est maintenue au reflux sous atmosphère d'azote pendant 24 heures. Le diglyme est évaporé sous pression réduite et on ajoute 100 ml de HCl 2M. Le 2-difluorométhyl-4,5-dicyano imidazole est extrait par trois portions d'éther de 30 ml. Après évaporation des extraits combinés, le produit est purifié par sublimation sous vide à 115°C sous vide primaire. Le sel de lithium est comme dans les exemples précédents, obtenu à partir du carbonate de lithium en léger excès dans l'acétonitrile

### Exemple 5

On a préparé l'ester méthylique de l'acide 3,3,3-trifluorométhoxy-2-fluoro-2-méthoxy propano que par condensation de 16 g d'époxy-hexafluoropropène C₃F₆O dans 75 ml de méthanol anhydre à -30°C . On sépare l'ester par dilution par l'eau, extraction au dichlorométhane et distillation. On hydrolyse 8,5 g de CF₃C(OCH₃)FCC(=O)OCH₃ par 2,4 g d'hydroxyde de sodium dans l'éthanol, on évapore le solvant et on reprend le solide par l'acétonitrile dans lequel seul CF₃C(OCH₃)FCCO₂Na est soluble. Ledit sel est ensuite séparé par filtration et évaporation de l'éthanol.

On fait réagir 9,9 g dudit sel de sodium, et 4,95 g de triphosgène (CCl₃O)₂C=O en présence de 50 mg de diméthylformamide (DMF) comme catalyseur dans le dioxane à 0°C. On ajoute 5,40 g de DAMN et le mélange est porté à reflux sous atmosphère d'azote pendant 24 heures. L'imidazole A5 est transformé en sel de lithium B5 par action du carbonate de lithium.

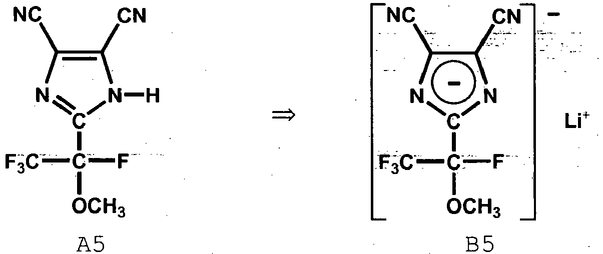

### Exemple 6

Un liquide ionique est préparé par action de 3,84 g du sel le lithium de l'exemple 1 sur 4,75 g d'éthylsulfate d'éthyl-méthyl-imidazolium N₂O₄SC₈H₁₆ dans 30 ml d'eau. Le liquide ionique qui se sépare est extrait au dichlorométhane et lavé à trois reprises par l'eau. Après évaporation du solvant, on obtient une huile fluide qui correspond à la formule :

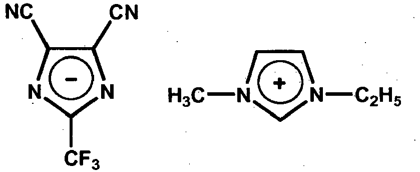

Ce fluide n'a pas de pression de vapeur détectable et il est stable jusqu'à 375°C.

### Exemple 7

L'ester 2-méthoxyéthylique de l'acide di-fluoro-(2-méthoxy-éthoxy)-propano que est obtenu par condensation de 16,6 g d'époxy-tétrafluoroéthylene C₂F₄O dans 250 ml de méthoxyéthanol anhydre à -30°C.

L'ester CH₃O-C₂H₄O-F₂C-C(=O)-OCH₃ est séparé par distillation. On hydrolyse 11,4 g d'ester par 3 g d'hydroxyde de potassium dans l'éthanol, on évapore le solvant et le méthoxyéthanol résultant, puis le solide après séchage sous vide à 70°C. Le solide est repris par l'acétonitrile dans lequel seul le sel de potassium (CH₃O-C₂H₄O-CF₂-CO₂K est soluble. Ce sel est récupéré par filtration et évaporation.

On fait réagir dans 35 ml de diglyme à 0°C, 6,6 g de sel de sodium, 4 g de chlorure de thionyle SOCl₂ et 50 mg de diméthylformamide (DMF) comme catalyseur. Après 1 h, on ajoute 3,6 g de DAMN et-le mélange est porté à reflux sous atmosphère d'azote pendant 24 heures. L'imidazole A7, extrait et purifié comme à l'exemple 5, est transformé en sel de lithium B7 par action du carbonate de lithium.

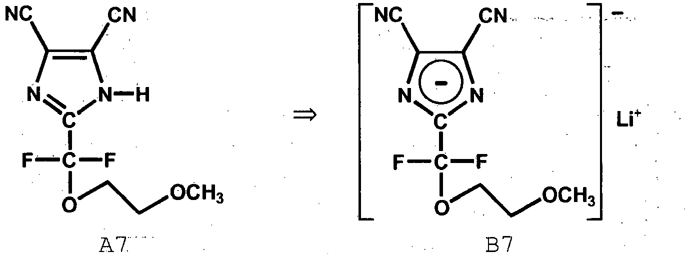

### Exemple 8

Le bromure de butyl-pyridinium est préparé par réaction de Menshutkin de 27,5 g de 1-bromobutane sur 15,8 g de pyridine à 40°C en 24 heures, sans solvant. Le solide obtenu est séché sous vide primaire à 50°C.

Un liquide ionique est préparé par action 4,84 g du sel de lithium de l'exemple 2 (CN)₂C₃N₂C₂F₅Li sur 4,35 g de bromure de butyl-pyridinium dans 25 ml d'eau. Le liquide ionique qui se sépare est extrait au dichlorométhane et lavé à trois reprises par l'eau. Après évaporation du solvant, on obtient une huile fluide qui correspond à la formule

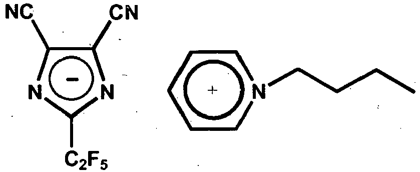

Ce fluide n'a pas de pression de vapeur détectable et est stable jusqu'à 375°C.

### Exemple 9

Le bromure de propyl-méthyl-pyrrolidinium est préparé par réaction de 12,4g de 1-bromopropane sur 8,5 g de N-métlylpyrrolidine à température ordinaire.

Un liquide ionique est préparé par action de 3,84 g du sel (CN)₂C₃N₂CF₃Li obtenu selon l'exemple 1 sur 4,18 g de bromure de propyl-méthyl-pyrrolidinium dans 25 ml d'eau. Le liquide ionique qui se sépare est extrait au dichlorométhane et lavé à trois reprises par l'eau. Après évaporation du solvant, on obtient une huile fluide qui correspond à la formule

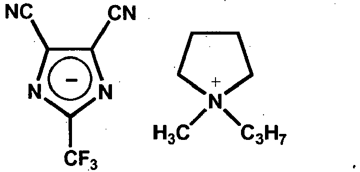

Ce fluide n'a pas de pression de vapeur détectable et est stable jusqu''a 375°C.

### Exemple 10

18 g d'hémiacétal de l'aldéhyde pentafluoropropionique C₂F₅CH(OH)OCH₃ commercial sont ajoutés à 10,8 g de DAMN dans 50 ml d'acétonitrile. Le mélange est maintenu à 50°C sous agitation pendant 24 heures. Ensuite, le mélange réactionnel est refroidi à -10°C et on ajoute goutte à goutte 16 g de brome dans l'acétonitrile. Le solvant est évaporé. Le 2-pentafluoroéthyl-4,5-dicyanoimidazole brut est purifié par sublimation à 100°C sous vide dans un four Büchi. Le sel de lithium est préparé comme précédemment par action du carbonate de lithium.

### Exemple 11

On a comparé la conductivité du sel de lithium LiTDCI de l'exemple 1 et du sel LiPDCI de l'exemple 2 à celle de divers sels de l'art antérieur connus pour les batteries au lithium. Les mesures ont été effectuées à partir d'une solution 1M de chaque sel dans un mélange carbonate d'éthylène-carbonate de méthyle (EC-DMC) 50/50 v/v, à 20°C.

| Sel | conductivité (mS.cm⁻¹) |
|---|---|
| LiPF₆ | 10,.8 |
| LiTFSI | 9,0 |
| LiTDCI | 6,7 |
| LiPDCI | 6,3 |
| LiDCTA | 2,9 |

Ce tableau montre que les performances de LiTDCI et de LiPDCI sont nettement meilleures que celles de LiDCTA, la conductivité étant plus que doublée.

### Exemple 12

On a assemblé trois batteries du type "Swagelok" Li/électrolyte Sel 1 M dans EC-DMC/LiFePO₄ comprenant une anode de lithium, un électrolyte liquide constitué par une solution 1M d'un sel dans un mélange EC/DMC 50/50, et une cathode constituée par un mélange de LiFePO₄ contenant 15% en masse de carbone SP sur un collecteur de Pt.

Le sel est respectivement le sel LiTDCI de l'exemple 1, le sel LiPDCI de l'exemple 2 et, à titre comparatif, le sel LiPF₆.

L'aptitude à conserver sa capacité en fonction de la puissance demandée a été vérifiée pour chacune des batteries selon le processus suivant. On a fait fonctionner chaque batterie plusieurs fois, avec un courant imposé différent, à 22°C, et on a noté la durée nécessaire pour obtenir la décharge totale en fonction de la durée théorique nécessaire pour une décharge totale. Les résultats sont représentés par les courbes de Ragone de la figure 2, sur laquelle "%C" en ordonnée indique le pourcentage de capacité restant, en fonction de la vitesse de décharge t(xc) indiquée en abscisse. T(xc) représente l'inverse du temps, en heures.

Ces courbes montrent que les batteries dans lesquelles le sel de l'électrolyte est un composé ionique tel que défini dans la composition d'électrolyte de l'invention ont une performance comparable à celle d'une batterie dans laquelle l'électrolyte est LiPF₆ considéré comme l'un des sels les plus conducteurs couramment utilisé dans les batteries au lithium à électrolyte liquide.

### Exemple 13

On a assemblé des batteries analogues à celles de l'exemple 12, en utilisant un collecteur de courant en aluminium pour la cathode en vue de tester la résistance à la corrosion de l'aluminium en fonction de diverses compositions d'électrolyte.

Le sel est respectivement le sel LiTDCI de l'exemple 1, le sel LiPDCI de l'exemple 2 et, à titre comparatif, le sel LiPF₆ et le sel LiTFSI.

On a fait soumis chaque batterie à une voltamétrie cyclique avec un régime de 10 mV/min.

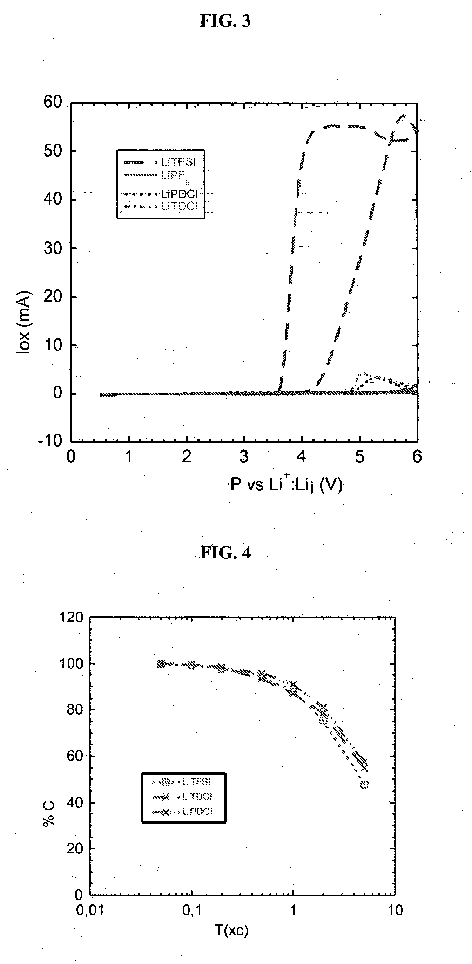

Les résultats sont reportés sur la figure 3 sur laquelle le courant d'oxydation Iₒₓ en mA est donnée en ordonnée, en fonction de P (potentiel vs Li⁺/Li) en Volts.

Comme attendu, LiPF₆ ne donne pas de corrosion appréciable, et LiTFSI au contraire s'avère très corrosif. Les sels de la composition d'électrolyte de l'invention LiTDCI et LiPDCI ne donnent pas de corrosion avant leur oxydation à 4,6 V vs. Li⁺ : Li°. Il est rappelé que la plupart des matériaux d'électrode de type oxyde ou Li₁₋ₓFeₓPO₄ finissent leur recharge à 4.3 V vs. Li⁺ : Li°, ce qui montre l'intérêt des composé ioniques utilisés de la composition d'électrolyte de l'invention qui ne corrodent pas l'aluminium à ce potentiel.

### Exemple 14

On a assemblé trois batteries du type "pile bouton" Li/électrolyte sel+POE /LiFePO₄ comprenant une anode de lithium, un électrolyte polymère constitué par une solution solide d'un sel dans un poly(oxyéthylène) POE, et une électrode positive constituée par un mélange de 40 % LiFePO₄, 10% de carbone SP et 50% de PEO en fraction massique sur un collecteur en acier inoxydable.

Chacun des électrolytes est préparé selon le mode opératoire de l'exemple 1, pour former des films de ≈ 100 µm d'épaisseur, en utilisant des quantités de polymère et de sel de lithium pour obtenir un rapport O/Li = 20.

Le sel est respectivement le sel LiTDCI de l'exemple 1, le sel LiPDCI de l'exemple 2 et, à titre comparatif, le sel LiTFSI.

L'aptitude à conserver sa capacité en fonction de la puissance demandée a été vérifiée pour chacune des batteries selon le processus suivant. On a fait fonctionner chaque batterie plusieurs fois, avec un courant imposé différent, à 80°C, et on a noté la durée nécessaire pour obtenir la décharge totale en fonction de la durée théorique nécessaire pour une décharge totale. Les résultats sont représentés par les courbes de Ragone de la figure 2, sur laquelle "%C" en ordonnée indique le pourcentage de capacité restant, en fonction de la vitesse de décharge t(xc) indiquée en abscisse. T(xc) représente l'inverse du temps, en heures.

Ces courbes montrent que les batteries dans lesquelles le sel de l'électrolyte est un composé ionique tel que défini dans la composition d'électrolyte de l'invention ont une performance comparable à celle d'une batterie dans laquelle l'électrolyte est LiTFSI considéré comme l'un des sels les plus conducteurs couramment utilisé dans les batteries au lithium à électrolyte polymère.

## Revendications

1. Composition d'électrolyte contenant un sel et un solvant, **caractérisée en ce que** le sel a un cation M de valence m (1≤m≤3) et m anions répondant à la formule : 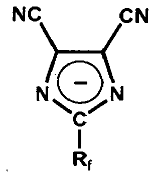 dans laquelle R_{f} est un groupe -CFZ'Z" dans lequel :
- Z' est F ou un groupe perfluoroalkyle ayant de 1 à 3 atomes de carbone,
- Z" est un groupe H, F, Cl, un groupe alcoxy éventuellement fluoré ou perfluoré ayant de 1 à 5 atomes de carbone, un groupe oxaalcoxy éventuellement fluoré ou perfluoré ayant de 1 à 5 atomes de carbone, ou un groupe alkyle éventuellement fluoré ou perfluoré ayant de 1 à 5 atomes de carbone,
le cation M étant un cation inorganique choisi parmi les cations de métal alcalin et les cations de métal alcalino-terreux ou un cation ammonium.

2. Composition d'électrolyte selon la revendication 1, **caractérisée en ce que** R_{f} est choisi dans le groupe constitué par CF₃, CF₂H, CF₂Cl, C₂F₅, CF₂CF₂H, C₃F₇, C₄F₉CF₂OCH₃, CF₂OC₂H₅, CF₂OC₂H₄OCH₃, CF₂OC₂H₄OC₂H₅, CF₂OCH₂OCF₃, CF(CF₃)OCH₃, CF(CF₃)OC₂H₅, CF(CF₃)OC₂H₄OCH₃, et CF(CF₃)OC₂H₄OC₂H₅.

3. Composition d'électrolyte selon la revendication 1, **caractérisée en ce que** le cation du sel de l'électrolyte est un ion lithium.

4. Composition d'électrolyte selon la revendication 1, **caractérisée en ce que** le solvant est choisi parmi les solvants organiques liquides éventuellement gélifiés par un polymère, les polymères solvatants éventuellement plastifiés par un solvant liquide, et les liquides ioniques.

5. Composition d'électrolyte selon la revendication 4, **caractérisée en ce que** le liquide ionique est un composé comprenant un cation M de valence m (1≤m≤3) et m anions répondant à la formule : 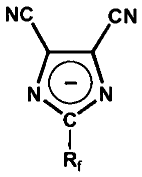 dans laquelle R_{f} est un groupe -CFZ'Z" dans lequel :
- Z' est F ou un groupe perfluoroalkyle ayant de 1 à 3 atomes de carbone,
- Z" est un groupe H, F, Cl, un groupe alcoxy éventuellement fluoré ou perfluoré ayant de 1 à 5 atomes de carbone, un groupe oxaalcoxy éventuellement fluoré ou perfluoré ayant de 1 à 5 atomes de carbone, ou un groupe alkyle éventuellement fluoré ou perfluoré ayant de 1 à 5 atomes de carbone ;
le cation M étant un cation organique, choisi parmi les ions ammonium, phosphonium, sulfonium, iodonium, pyridinium, imidazolium, pyrazolium, acetamidium, oxazolium, thiazolium, pyrrolidinium et piperidinium.

6. Composition d'électrolyte selon la revendication 5, **caractérisée en ce que** le cation du composé formant le liquide ionique est choisi parmi les les cations éthyl-méthyl-imidazolium, butyl-méthyl-imidazolium, méthyl-propyl-pyrrolidi-nium, méthyl-butyl-pyrrolidinium, méthyl-propyl-piperidinium, butyl pyridinium, (2-méthoxy-éthyl)-triéthyl-ammonium, et hexyl-trimethyl ammonium.

7. Composition d'électrolyte selon la revendication 4, **caractérisée en ce que** le solvant est un liquide polaire ou un mélange de liquides polaires, et la concentration en sel est de 10⁻³ mole/L à 3,5 mole/L.

8. Composition d'électrolyte selon la revendication 4, **caractérisée en ce que** le solvant est un polymère solvatant comprenant des unités récurrentes oxyalkylène, et la concentration en sel est telle que le nombre d'atomes d'oxygène (ou d'unités récurrentes) par mole de sel est entre 1 et 200.

9. Composition d'électrolyte selon la revendication 4, **caractérisée en ce que** le solvant est un liquide ionique et la concentration en sel est 10⁻³ mole/L à 3,5 mole/L.

## Patentansprüche

1. Elektrolytzusammensetzung, die ein Salz und ein Lösungsmittel enthält, **dadurch gekennzeichnet, dass** das Salz ein Kation M mit der Wertigkeit m (1≤m≤3) und m Anionen hat, entsprechend der Formel 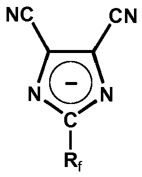 in welcher R_{f} eine Gruppe -CFZ'Z" ist, in welcher:
- Z' F oder eine Perfluoralkylgruppe mit 1 bis 3 Kohlenstoffatomen ist,
- Z" eine Gruppe H, F, Cl, eine eventuell fluorierte oder perfluorierte Alcoxygruppe mit 1 bis 5 Kohlenstoffatomen, eine eventuell fluorierte oder perfluorierte Oxaalcoxygruppe mit 1 bis 5 Kohlenstoffatomen oder eine eventuell fluorierte oder perfluorierte Alkylgruppe mit 1 bis 5 Kohlenstoffatomen ist,
wobei das Kation M ein anorganisches Kation, das aus den alkalischen Metallkationen und den erdalkalische Metallkationen ausgewählt ist, oder ein Ammoniumkation ist.

2. Elektrolytzusammensetzung nach Anspruch 1, **dadurch gekennzeichnet, dass** R_{f} aus der Gruppe ausgewählt ist, die von CF₃, CF₂H, CF₂Cl, C₂F₅, CF₂CF₂H, C₃F₇, C₄F₉CF₂OCH₃, CF₂OC₂H₅, CF₂OC₂H₄OCH₃, CF₂OC₂H₄OC₂H₅, CF₂OCH₂OCF₃, CF(CF₃)OCH₃, CF(CF₃)OC₂H₅, CF(CF₃)OC₂H₄OCH₃ und CF(CF₃)OC₂H₄OC₂H₅ gebildet ist.

3. Elektrolytzusammensetzung nach Anspruch 1, **dadurch gekennzeichnet, dass** das Kation des Salzes des Elektrolyts ein Lithiumion ist.

4. Elektrolytzusammensetzung nach Anspruch 1, **dadurch gekennzeichnet, dass** das Lösungsmittel aus den flüssigen organischen Lösungsmitteln, die eventuell durch ein Polymer geliert sind, den solvatisierenden Polymeren, die eventuell von einem flüssigen Lösungsmittel plastifiziert sind, und den ionischen Flüssigkeiten ausgewählt ist.

5. Elektrolytzusammensetzung nach Anspruch 4, **dadurch gekennzeichnet, dass** die ionische Flüssigkeit eine Verbindung ist, die ein Kation M mit der Wertigkeit m (1≤m≤3) und m Anionen umfasst, entsprechend der Formel 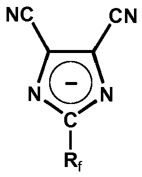 in welcher R_{f} eine Gruppe -CFZ'Z" ist, in welcher:
- Z' F oder eine Perfluoralkylgruppe mit 1 bis 3 Kohlenstoffatomen ist,
- Z" eine Gruppe H, F, Cl, eine eventuell fluorierte oder perfluorierte Alcoxygruppe mit 1 bis 5 Kohlenstoffatomen, eine eventuell fluorierte oder perfluorierte Oxaalcoxygruppe mit 1 bis 5 Kohlenstoffatomen oder eine eventuell fluorierte oder perfluorierte Alkylgruppe mit 1 bis 5 Kohlenstoffatomen ist,
wobei das Kation M ein organisches Kation ist, das aus den Ammonium-, Phosphonium-, Sulfonium, Iodonium-, Pyridinium-, Imidazolium-, Pyrazolium-, Acetamidium-, Oxazolium-, Thiazolium-, Pyrrolidinium- und Piperidinium-Ionen ausgewählt ist.

6. Elektrolytzusammensetzung nach Anspruch 5, **dadurch gekennzeichnet, dass** das Kation der Verbindung, die die ionische Flüssigkeit bildet, aus den Ethyl-methyl-imidazolium-, Butyl-methyl-imidazolium-, Methyl-propyl-pyrrolidinium-, Methylbutyl-pyrrolidinium-, Methyl-propyl-piperidinium-, Butylpyridinium-, (2-Methoxy-ethyl)-triethyl-ammonium- und Hexyl-trimethyl-ammonium-Kationen ausgewählt ist.

7. Elektrolytzusammensetzung nach Anspruch 4, **dadurch gekennzeichnet, dass** das Lösungsmittel eine polare Flüssigkeit oder ein Gemisch polarer Flüssigkeiten ist und die Salzkonzentration 10⁻³ mol/L bis 3,5 mol/L beträgt.

8. Elektrolytzusammensetzung nach Anspruch 4, **dadurch gekennzeichnet, dass** das Lösungsmittel ein solvatisierendes Polymer ist, das rekurrente Oxyalkyleneinheiten umfasst und die Salzkonzentration derart ist, dass die Anzahl von Sauerstoffatomen (oder von rekurrenten Einheiten) je Mol Salz zwischen 1 und 200 ist.

9. Elektrolytzusammensetzung nach Anspruch 4, **dadurch gekennzeichnet, dass** das Lösungsmittel ein ionisches Lösungsmittel ist und die Salzkonzentration 10⁻³ mol/L bis 3,5 mol/L ist.

## Claims

1. An electrolyte composition containing a salt and a solvent **characterized in that** the salt has a cation M of valence m (1≤m≤3) and m anions meeting formula: 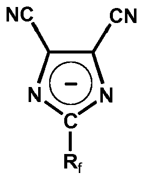 where R_{f} is a -CFZZ" group in which:
- Z' is F or perfluoroalkyl group having 1 to 3 carbon atoms;
- Z" is a group H, F or Cl, an alkoxy group optionally fluorinated or perfluorinated having 1 to 5 carbon atoms, an oxaalkoxy group optionally fluorinated or perfluorinated having 1 to 5 carbon atoms, or an alkyl group optionally fluorinated or perfluorinated having 1 to 5 carbon atoms;
the cation M being an inorganic cation selected from among cations of alkaline metals and cations of alkaline-earth metals or an ammonium cation.

2. The electrolyte composition according to claim 1, **characterized in that** R_{f} is selected from the group formed by CF₃, CF₂H, CF₂Cl, C₂F₅, CF₂CF₂H, C₃F₇, C₄F₉CF₂OCH₃, CF₂OC₂H₅, CF₂OC₂H₄OCH₃, CF₂OC₂H₄OC₂H₅, CF₂OCH₂OCF₃, CF(CF₃)OCH₃, CF(CF₃)OC₂H₅, CF(CF₃)OC₂H₄OCH₃ and CF(CF₃)OC₂H₄OC₂H₅.

3. The electrolyte composition according to claim 1 **characterized in that** the cation of the electrolyte salt is a lithium ion.

4. The electrolyte composition according to claim 1, **characterized in that** the solvent is selected from among liquid organic solvents optionally gelled by a polymer, solvating polymers optionally plasticised by a liquid solvent and ionic liquids.

5. The electrolyte composition according to claim 4, **characterized in that** the ionic liquid is a compound comprising a cation M of valence m (1≤m≤3) and m anions meeting formula: 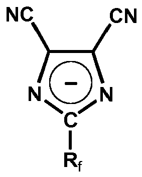 where R_{f} is a -CFZ'Z" group in which:
- Z' is F or perfluoroalkyl group having 1 to 3 carbon atoms;
- Z" is a group H, F or Cl, an alkoxy group optionally fluorinated or perfluorinated having 1 to 5 carbon atoms, an oxaalkoxy group optionally fluorinated or perfluorinated having 1 to 5 carbon atoms, or an alkyl group optionally fluorinated or perfluorinated having 1 to 5 carbon atoms;
the cation M being an organic cation selected from among the following ions: ammonium, phosphonium, sulfonium, iodinium, pyridinium, imidazolium, pyrazolium, acetamidium, oxazolium, thiazolium, pyrrolidinium and piperidinium.

6. The electrolyte composition according to claim 5, **characterized in that** the cation of the compound forming the ionic liquid is selected from among the following cations: ethyl-methyl-imidazolium, butyl-methyl-imidazolium, methyl-propyl-pyrrolidinium, methyl-butyl-pyrrolidinium, methyl-propyl-piperidinium, butyl pyridinium, (2-methoxy-ethyl)-triethyl-ammonium and hexyl-trimethyl ammonium.

7. The electrolyte composition according to claim 4, **characterized in that** the solvent is a polar liquid or mixture of polar liquids and the salt concentration is 10⁻³ mole/L to 3.5 mole/L.

8. The electrolyte composition according to claim 4, **characterized in that** the solvent is a solvating polymer comprising repeating oxyalkyene units and the salt concentration is such that the number of oxygen atoms (or repeating units) per mole of salt is between 1 and 200.

9. The electrolyte composition according to claim 4, **characterized in that** the solvent is an ionic liquid and the salt concentration is 10⁻³ mole/L to 3.5 mole/L.
